(19) Europäisches Patentamt
European Patent Office
Office européen des brevets

(11) Veröffentlichungsnummer : **0 054 146**
**B1**

# (12) EUROPÄISCHE PATENTSCHRIFT

(45) Veröffentlichungstag der Patentschrift : **18.01.84**

(51) Int. Cl.³ : **C 12 Q 1/00**, C 12 Q 1/26

(21) Anmeldenummer : **81108756.8**

(22) Anmeldetag : **22.10.81**

## (54) Nachweis von NAD (P) H oder Salicylat.

(30) Priorität : **11.12.80 DE 3046741**

(43) Veröffentlichungstag der Anmeldung : **23.06.82 Patentblatt 82/25**

(45) Bekanntmachung des Hinweises auf die Patenterteilung : **18.01.84 Patentblatt 84/03**

(84) Benannte Vertragsstaaten :
**AT BE CH DE FR GB IT LI LU NL SE**

(56) Entgegenhaltungen :
**EP-A- 0 029 104**
**WO-A-81 /007 25**
**DE-A- 1 959 410**
**CHEMICAL ABSTRACTS, Band 76, Nr. 21, 22. Mai 1972, Seite 205, Nr. 123427w, Columbus, Ohio, USA, R.H. WHITE-STEVENS et al.: "Flavoprotein, salicylate hydroxylase. I. Preparation, properties, and the uncoupling of oxygen reduction from hydroxylation"**
**CHEMICAL ABSTRACTS, Band 78, Nr. 17, 30. April 1973, Seite 162, Nr. 107424d, Columbus, Ohio, USA, P.G. PIFFERI et al.: "Spectrophotometric method for the determination of the catecholase activity of tyrosinase by Besthorn's hydrazone"**

(73) Patentinhaber : **BOEHRINGER MANNHEIM GMBH**
**Patentabteilung, Abt. E Sandhofer Strasse 112-132**
**Postfach 31 01 20**
**D-6800 Mannheim 31 Waldhof (DE)**

(72) Erfinder : **Röder, Albert, Dr.**
**Bahnhofstrasse 41**
**D-8124 Seeshaupt (DE)**
Erfinder : **Siedel, Joachim, Dr.**
**Bahnhofstrasse 6**
**D-8131 Bernried (DE)**
Erfinder : **Möllering, Hans**
**Herrestrasse 10**
**D-8132 Tutzing (DE)**
Erfinder : **Seidel, Hans, Dr.**
**Waxensteinstrasse 6**
**D-8132 Tutzing (DE)**
Erfinder : **Gauhl, Helmgard**
**Kustermannstrasse 31**
**D-8132 Tutzing (DE)**

(74) Vertreter : **Weickmann, Heinrich, Dipl.-Ing. et al**
**Patentanwälte Dipl.-Ing. H. Weickmann Dipl.-Phys.Dr. K. Fincke Dipl.-Ing. F.A. Weickmann Dipl.-Chem. B. Huber Dr.-Ing. H. Liska Möhlstrasse 22**
**D-8000 München 86 (DE)**

**EP 0 054 146 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Nachweis von NAD(P)H oder Salicylat

Die Erfindung betrifft ein Verfahren zum Nachweis von NAD(P)H oder NAD(P)H bildenden Enzymreaktionen oder von Salicylat bzw. von Salicylat liefernden Enzymreaktionen.

Die Bestimmung diagnostischer Parameter, wie Glucose oder Glycerin, ist von großer Bedeutung für die klinische Chemie. Üblicherweise werden dazu Dehydrogenasen oder Oxidasen eingesetzt.

Oxidasen werden bevorzugt verwendet, da das entstehende $H_2O_2$ durch eine Farbreaktion nachgewiesen werden kann, indem man z. B. in Gegenwart von Peroxidase Chromogene unter Einwirkung von $H_2O_2$ oxidativ zu einem Farbstoff kuppelt. Derartige Systeme haben den Vorteil, daß die zur Bestimmung verwendeten Reagenzien stabil sind und die Möglichkeit bieten, durch geeignete Auswahl der Kupplungspartner Extinktionsmaximum und Wellenlänge so zu beeinflussen, daß störungsfrei gemessen werden kann.

Jedoch weist diese Methode auch schwerwiegende Nachteile auf : So sind Oxidasen verhältnismäßig unspezifische Enzyme, so daß sich die Anwesenheit anderer oxidierbarer Substanzen im Testansatz als störend erweisen kann. Ausserdem wird das Intermediärprodukt $H_2O_2$ häufig nicht stöchiometrisch gebildet und ist zudem sehr instabil. Die Umsetzung des gebildeten $H_2O_2$ durch Peroxidase verläuft stöchiometrisch ungünstig : Es werden mindestens 2 Mol $H_2O_2$ pro Mol entstehendem Farbstoff benötigt.

Hingegen verlaufen Dehydrogenase-Reaktionen streng stöchiometrisch und im allgemeinen auch wesentlich spezifischer als Oxidasereaktionen. Außerdem ist das Intermediärprodukt NAD(P)H sehr stabil.

Es wäre deshalb wünschenswert, die Vorteile der spezifischen und stöchiometrischen Dehydrogenasereaktion mit den Vorteilen einer farbstoffbildenden Reaktion zu verbinden.

Es ist bekannt, daß das Reaktionsprodukt NAD(P)H in einer Dehydrogenase-Reaktion mittels Diaphorase und Tetrazoliumsalzen in einen Farbstoff konvertiert werden kann. Jedoch weist dieses System ebenfalls große Unzulänglichkeiten auf, da Tetrazoliumsalze instabil sind und der gebildete Farbstoff zur Polymerisation neigt und damit unlöslich wird.

Der Erfindung liegt daher die Aufgabe zugrunde, ein Verfahren zur Visualisierung von NAD(P)H zu schaffen, welches diese Nachteile nicht aufweist und auch den Bedarf nach einer Salicylat-Bestimmungsmethode befriedigt.

Gelöst wird diese Aufgabe erfindungsgemäß durch ein Verfahren zur Bestimmung von NAD(P)H oder Salicylat, welches dadurch gekennzeichnet ist, daß man in einer NAD(P)H-abhängigen Reaktion Salicylat durch Salicylathydroxylase decarboxyliert und aus dem Decarboxylierungsprodukt in Gegenwart von Tyrosinase durch oxidative Kupplung mit einer geeigneten Farbstoffkomponente einen Farbstoff bildet, welcher photometrisch bestimmt wird.

Dem erfindungsgemäßen Verfahren liegen folgende bekannte Reaktionen zugrunde :

(1) Salicylat + NAD(P)H + $O_2$

$$\xrightarrow{\text{Salicylat-hydroxylase (E.C. 1.14.13.1)}} \text{Brenzkatechin} + CO_2 + H_2O + NAD^+$$

(2) Brenzkatechin + Hydrazon + $O_2$

$$\xrightarrow{\text{Tyrosinase (E.C. 1.10.3.1) oder (E.C. 1.14.18.1)}} \text{Acin-Farbstoff} + H_2O$$

Reaktion (1) ist bekannt aus R. H. White-Stevens und H. Kamin, J. Biol. Chem. *247*, 2358 (1972). Unter Salicylat werden außer Salicylat alle Salicylat-Derivate verstanden.

Von Reaktion (2) ist bekannt, daß Tyrosinase außer Brenzkatechin auch Monophenole oxidiert und an die dabei entstehenden Chinone Hydrazone oxidativ zu Farbstoffen kuppelt (C.R. Dawson und R.J. Magee, Methods in Enzymology *2*, 817 (1955) ; B.G. Malmström und L. Ryden, Biological Oxidations ; (Singer T. P. Ed.), S. 419 Interscience Publ. N. Y. (1968). Daher war nicht zu erwarten, daß eine Kopplung der Reaktionen (1) und (2) als Indikatorsystem für NAD(P)H geeignet wäre, da eine Störung durch direkte Reaktion von Salicylat mit dem Hydrazon in Gegenwart der Tyrosinase zu erwarten war.

Überraschenderweise konnte jedoch nun gezeigt werden, daß Tyrosinase Salicylat nicht umsetzt und lediglich das Reaktionsprodukt Brenzkatechin unter den Bedingungen des Tests oxidativ kuppelt. Diese Reaktion erfolgt stöchiometrisch, wobei pro Mol Brenzkatechin 1 Mol eines stabilen Farbstoff-Komplexes entsteht.

Die Erfindung besitzt ein breites Anwendungsgebiet für alle NADH- oder NADPH-liefernden Reaktionen. Als NAD(P)H-bildende Reaktion kommt z. B. die Bestimmung von Substraten, wie Glucose, Glucose-6-phosphat, Glycerin und Triglyceriden mit Hilfe verschiedener Dehydrogenasen in Betracht. Auch die Bestimmung von Enzymaktivitäten verschiedener Dehydrogenasen, wie beispielsweise Glucose-6-phosphat-dehydrogenase, ist möglich. Aus den dem Verfahren zugrunde liegenden Reaktionen ergibt sich gleichzeitig auch die Möglichkeit, die Erfindung auch zur Bestimmung von Salicylat oder Salicylat-Derivaten zu verwenden, wenn gegenüber dem zu bestimmenden Salicylat NAD(P)H im Überschuß im Reaktionsgemisch vorliegt. Auf diese Weise können alle salicylatbildenden Reaktionen bestimmt werden. Eine Möglichkeit ist. z. B. die Bestimmung von Hydrolasen, die Verbindungen der Formel

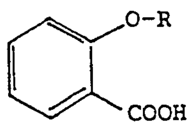

oder

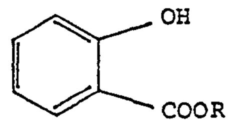

zu spalten vermögen, darunter Proteasen, Gerinnungsfaktoren, Amylase und Lipase.

Als Farbstoffbildungskomponente kommen chromogene Hydrazone und Amine in Betracht.

Durch Auswahl verschiedener kupplungsfähiger Hydrazone bzw. Amine können Extinktion und Wellenlänge nach Bedarf beeinfluß werden. Geeignete Verbindungen sind z. B. alle Hydrazone, die von S. Hünig in Angewandte Chemie *70*, 215 (1958) beschrieben sind. In einer bevorzugten Ausführungsform wird als kupplungsfähiges Hydrazon 3-Methyl-2-benzo(2'-sulfo)-thiazolon-hydrazon (MBTH-S) verwendet.

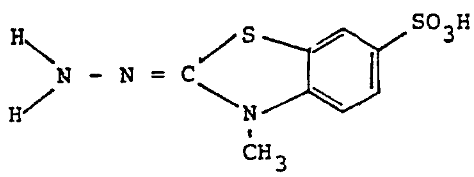

MBTH-S

Als chromogene Amine eignen sich z. B. Prolin, Protamin, Histamin und Lysin.

Gegenstand der Erfindung ist weiter ein Reagenz zur Bestimmung von NADH oder NADPH, welches dadurch gekennzeichnet ist, daß es Salicylat, ein chromogenes Hydrazon oder Amin, Salicylathydroxylase, Tyrosinase und Puffer enthält ; und ein Reagenz zur Bestimmung von Salicylat, welches dadurch gekennzeichnet ist, daß es NAD(P)H, chromogenes Hydrazon oder Amin, Salicylathydroxylase, Tyrosinase und Puffer enthält. Die Reagenzkombination können außer den aufgeführten obligaten Bestandteilen zusätzlich übliche Lösungsmittel und Hilfsmittel, wie z. B. Stabilisatoren und grenzflächenaktive Substanzen enthalten.

Geeignet sind alle Puffer, die in einem pH-Bereich von 6 bis 9,3 puffern, mit einer Pufferstärke von 0,005 bis 1,0 mol/l. Bevorzugt werden Phosphat- oder Glycylglycin-Puffer. Die Enzyme werden zweckmäßig in folgenden Konzentrationen eingesetzt ; Salicylathydroxylase 0,05 bis 5,0 kU/l, Tyrosinase 10 bis 200 kU/l. Das erfindungsgemäße Reagens kann auch auf Trägern, wie z. B. Papier, Kunststoffolien oder anderen porösen Körpern imprägniert vorliegen und so z. B. als Teststreifen Verwendung finden.

Die folgenden Beispiele erläutern die Erfindung weiter.

## Beispiel 1

NADH-Bestimmung

Das Reagens besteht aus :

| | |
|---|---|
| MBTH-S | 1,7 mmol/l |
| SAHX | 70 U/l |
| Tyrosinase | $33 \cdot 10^3$ U/l |
| Salicylat-Na | 0,33 mmol/l |
| K-Phosphat-Puffer pH 7,0 | 0,1 mol/l |
| Start der Reaktion : | Zugabe der NADH-haltigen Probe |
| Messung bei : | 492 nm |
| | 25 °C |
| Endpunkt : | nach 15 min. für 0,02 μmol NADH in 3 ml Testvolumen |

## Beispiel 2

NADPH-Bestimmung

Analog Beispiel 1

| | |
|---|---|
| Start der Reaktion : | Zugabe der NADPH-haltigen Probe |

## Beispiel 3

Bestimmung von Glucose-6-phosphat

Das Reagens besteht aus :

| | |
|---|---|
| MBTH-S<br>SAHX<br>Tyrosinase<br>Salicylat-Na<br>K-Phosphatpuffer pH 7,0 | } wie Beispiel 1 |
| Glucose-6-phosphat-dehydrogenase (leuconostoc mesenteroides) | $1{,}6 \cdot 10^3$ U/l |
| $NAD^+$ | 0,25 mmol/l |
| Start der Reaktion : | mit Probe |
| Messung bei : | 492 nm<br>25 °C |
| Endpunkt der Reaktion : | 5 min. |

## Beispiel 4

Bestimmung der Aktivität von Glucose-6-phosphatdehydrogenase

Das Reagens besteht aus :

| | |
|---|---|
| MBTH-S<br>SAHX<br>Tyrosinase<br>Salizylat-Na<br>K-Phosphatpuffer pH 7,0 | } wie Beispiel 1 |
| $NAD^+$ | 0,25 mmol/l |
| Glucose-6-phosphat | 3,3 mmol/l |
| Start : | mit der G-6-PDH-haltigen Probe |
| Messung bei : | 492 nm<br>25 °C |

## Beispiel 5

Glycerin-Bestimmung

Das Reagens besteht aus :

| | |
|---|---|
| MBTH-S | 1,7 mmol/l |
| SAHX | 80 U/l |
| Tyrosinase | $66 \cdot 10^3$ U/l |
| Glycerindehydrogenase | 2 400 U/l |
| Salizylat-Na | 0,33 mmol/l |
| $NAD^+$ | 1,35 mmol/l |
| Glycylglycin-Puffer pH 8,5 | 0,1 mol/l |
| $(NH_4)_2SO_4$ | 0,01 mol/l |
| Start : | durch Probezugabe |
| Messung : | 492 nm<br>25 °C |
| Endpunkt : | nach 30 min. |

## Beispiel 6

Bestimmung von Triglyceriden

Das Reagens besteht aus :

| | |
|---|---|
| MBTH-S<br>SAHX<br>Tyrosinase<br>Glycerindehydrogenase<br>Salizylat-Na<br>$NAD^+$<br>Glycylglycin-Puffer pH 8,5 $(NH_4)_2SO_4$ | wie Beispiel 5 |
| Esterase aus pseudomonas | 1 000 U/l |
| Isotridecyläther | 2 g/l |
| Start : | durch Probezugabe |
| Messung : | 492 nm<br>25 °C |
| Endpunkt : | nach 30 min. |

Beispiel 7

Bestimmung von Salicylsäure

Das Reagens besteht aus :

| | |
|---|---|
| MBTH-S | 1,7 mmol/l |
| SAHX | 70 U/l |
| Tyrosinase | $33 \cdot 10^3$ U/l |
| NADH | 0,23 mmol/l |
| K-Phosphatpuffer pH 7,0 | 0,1 mol/l |
| Start : | durch Probezugabe |
| Messung : | 492 nm<br>25 °C |
| Endpunkt : | nach 10 min. für 0,02 µmol Salicylat in 3 ml Testvolumen |

In den Beispielen 1 bis 7 kann jeweils MBTH-S in der Konzentration von 1,7 mmol/l durch 10 mmol/l Prolin ersetzt werden, ohne daß sich die Resultate ändern. Die Messung erfolgt in diesem Fall bei 546 nm.

**Ansprüche**

1. Verfahren zur Bestimmung von NAD(P)H oder Salicylat, dadurch gekennzeichnet, daß man in einer NADH(P)H-abhängigen Reaktion Salicylat durch Salicylathydroxylase decarboxyliert und aus dem Decarboxylierungsprodukt in Gegenwart von Tyrosinase durch oxidative Kupplung mit einer geeigneten Farbstoffkomponente einen Farbstoff bildet, welcher photometrisch bestimmt wird.

2. Reagenz zur Bestimmung von NADH oder NAD(P)H, dadurch gekennzeichnet, daß es Salicylat, ein chromogenes Hydrazon oder Amin, Salicylathydroxylase, Tyrosinase und Puffer enthält.

3. Reagenz zur Bestimmung von Salicylat, dadurch gekennzeichnet, daß es NAD(P)H, Hydrazon oder Amin, Salicylathydroxylase, Tyrosinase und Puffer enthält.

4. Reagenz nach Anspruch 2, dadurch gekennzeichnet, daß es 0,05 bis 20 mmol/l Salicylat, 0,3 bis 30 mmol/l Hydrazon oder Amin, 0,05 bis 5 kU/l Salicylathydroxylase, 10 bis 200 kU/l Tyrosinase und Puffer, pH 6,0 bis 9,3 enthält.

5. Reagenz nach Anspruch 3, dadurch gekennzeichnet, daß es 0,05 bis 1,5 mmol/l NAD(P)H, 0,3 bis 30 mmol/l Hydrazon oder Amin, 0,05 bis 5 kU/l Salicylathydroxylase, 10 bis 200 kU/l Tyrosinase und Puffer, pH 6,0 bis 9,3, enthält.

6. Reagenz nach Anspruch 2 bis 5, dadurch gekennzeichnet, daß es als Hydrazon 3-Methyl-2-benzo-(2′-sulfo)-thiazolon-hydrazon (MBTH-S) enthält.

**Claims**

1. Process for the determination of NAD(P)H or salicylate, characterised in that, in an NAD(P)H-dependent reaction, one decarboxylates salicylate by salicylate hydroxylase and forms a coloured material from the decarboxylation product in the presence of tyrosinase by oxidative coupling with a suitable coloured material component, which coloured material is determined photometrically.

2. Reagent for the determination of NADH or NAD(P)H, characterised in that it contains salicylate, a chromogenic hydrazone or amine, salicylate hydroxylase, tyrosinase and buffer.

3. Reagent for the determination of salicylate, characterised in that it contains NAD(P)H, hydrazone or amine, salicylate hydroxylase, tyrosinase and buffer.

4. Reagent according to claim 2, characterised in that it contains 0.05 to 20 mmol/l salicylate, 0.3 to

30 mmol/l hydrazone or amine, 0.05 to 5 kU/l salicylate hydroxylase, 10 to 200 kU/l tyrosinase and buffer, pH 6.0 to 9.3.

5. Reagent according to claim 3, characterised in that it contains 0.05 to 1.5 mmol/l NAD(P)H, 0.3 to 30 mmol/l hydrazone or amine, 0.05 to 5 kU/l salicylate hydroxylase, 10 to 200 kU/l tyrosinase and buffer, pH 6.0 to 9.3.

6. Reagent according to claims 2 to 5, characterised in that, as hydrazone, it contains 3-methyl-2-benzo-(3'-sulpho)-thiazolone hydrazone (MBTH-S).

**Revendications**

1. Procédé pour la détermination du NAD(P)H ou du salicylate, caractérisé en ce que l'on décarboxyle le salicylate par la salicylatehydroxylase dans une réaction dépendante du NAD(P)H, et en ce qu'à partir du produit de décarboxylation, en présence de tyrosinase, par couplage oxydatif avec un composant de substance colorée approprié, on forme une substance colorée, laquelle est déterminée photométriquement.

2. Réactif pour la détermination du NADH ou NAD(P)H, caractérisé en ce qu'il contient du salicylate, une hydrazone ou amine chromogène, de la salicylatehydroxylase, de la tyrosinase et du tampon.

3. Réactif pour la détermination du salicylate, caractérisé en ce qu'il contient du NAD(P)H, de l'hydrazone ou de l'amine, de la salicylatehydroxylase, de la tyrosinase et du tampon.

4. Réactif selon la revendication 2, caractérisé en ce qu'il contient 0,05 à 20 mmole/l de salicylate, 0,3 à 30 mmole/l d'hydrazone ou d'amine, 0,05 à 5 kU/l de salicylatehydroxylase, 10 à 200 kU/l de tyrosinase et du tampon, pH 6,0 à 9,3.

5. Réactif selon la revendication 3, caractérisé en ce qu'il contient 0,05 à 1,5 mmole/l de NAD(P)H, 0,3 à 30 mmole/l d'hydrazone ou d'amine, 0,05 à 5 kU/l de salicylatehydroxylase, 10 à 200 kU/l de tyrosinase et du tampon, pH 6,0 à 9,3.

6. Réactif selon la revendication 2 à 5, caractérisé en ce qu'il contient en tant qu'hydrazone de la 3-méthyl-2-benzo-(2'-sulfo)-thiazolone-hydrazone (MBTH-S).